(19) Europäisches Patentamt / European Patent Office / Office européen des brevets

(11) **EP 4 772 174 A1**

(12) **EUROPEAN PATENT APPLICATION**
published in accordance with Art. 153(4) EPC

(43) Date of publication:
08.07.2026 Bulletin 2026/28

(21) Application number: 24859874.0

(22) Date of filing: 29.08.2024

(51) International Patent Classification (IPC):
*A61K 9/14* (2006.01)     *A61K 9/20* (2006.01)
*A61K 47/02* (2006.01)    *A61K 47/12* (2006.01)
*A61K 47/14* (2017.01)    *A61K 47/26* (2006.01)
*A61K 47/32* (2006.01)    *A61K 47/36* (2006.01)
*A61K 47/38* (2006.01)

(52) Cooperative Patent Classification (CPC):
A61K 9/14; A61K 9/20; A61K 47/02; A61K 47/12;
A61K 47/14; A61K 47/26; A61K 47/32;
A61K 47/36; A61K 47/38

(86) International application number:
PCT/JP2024/030882

(87) International publication number:
WO 2025/047845 (06.03.2025 Gazette 2025/10)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 30.08.2023 JP 2023140282

(71) Applicant: **Fuji Chemical Industries Co., Ltd.**
**Kamiichi-machi**
**Nakaniikawa-gun**
**Toyama 930-0355 (JP)**

(72) Inventors:
• **MIYADA, Keisuke**
  **Nakaniikawa-gun, Toyama 930-0355 (JP)**
• **NAKASHIMA, Yo**
  **Nakaniikawa-gun, Toyama 930-0355 (JP)**
• **OONUKI, Tetsuya**
  **Nakaniikawa-gun, Toyama 930-0355 (JP)**

(74) Representative: **Wächtershäuser & Hartz**
**Patentanwaltspartnerschaft mbB**
**Weinstraße 8**
**80333 München (DE)**

(54) **CO-PROCESSED EXCIPIENT**

(57) Provided are a co-processed excipient which has favorable flowability, suppresses spread caused by the mixing of a tableting powder, suppresses delay in disintegration and reduction in compacting and hardness, and is used for producing a tablet improved in content uniformity, and a tablet comprising the same. The present invention provides a particulate composition comprising a lubricant, a low soluble saccharide, and anhydrous dibasic calcium phosphate.

**EP 4 772 174 A1**

**Description**

Technical Field

The present invention relates to a co-processed excipient which is excellent in disintegrating property and tabletability, suppresses spreadability, and is suitable for direct compression, a method for producing the excipient, and a tablet containing the excipient.

Background Art

Methods for producing tablets include, for example, a granule compression method of adding additives after wet granulation or dry granulation of an active ingredient, and mixing the additives and the granules, followed by tableting, and a direct tableting method of mixing all components without the active ingredient granulation step, followed by tableting. The direct tableting method has the advantage of requiring a smaller number of steps, such as a granulation step, in production and reducing production cost as compared with the granule compression method, or the advantage of being useful for an active ingredient vulnerable to water or heat because of the absence of the step of using water or a heating step. However, this method is disadvantageous in difficult design of content uniformity, compactibility, and an eluting property.

A method to manufacturing of tablets also include batch manufacturing and continuous manufacturing. The batch manufacturing is a manufacturing method where additives are mixed and granulated using the aforementioned granule compression method or direct compression method to prepare tableting powder in batches before tableting. This method has heretofore been frequently used. The continuous manufacturing is a manufacturing method that continuously mixes additives and granulates using an aforementioned granule compression method or direct compression method to prepare tableting powder, thereby enabling continuous tableting. Batch manufacturing allows sequential verification at each process step, making the manufacturing process itself easy to set up. However, when changing the batch size, such as from pilot scale to full scale, it was necessary to check mixability and other factors. In contrast, continuous manufacturing involves mixing and granulating each ingredient continuously. While designing the manufacturing process is challenging, changing the batch size only requires adjusting the production time. This eliminates scale-up obstacles and offers the advantage of lower manufacturing costs compared to batch processing during commercial production.

The addition of a lubricant is problematic in the preparation of the tableting powder mentioned above. In general, pharmaceutical additives components other than the lubricant are mixed and granulated, and the lubricant is then added and mixed immediately before tableting, followed by tableting. The lubricant causes spread during mixing the tableting powder and during flow in a hopper or on a turn table in tableting. Therefore, the status of addition is adjusted depending on each individual mixing machine or tableting machine. The lubricant is added for an effect of improving the releasability of tablets from a punch and a die after tableting, and eliminating tableting problems. The lubricant exerts its lubricating effect when present on tablet surface, whereas the lubricant present in the inside of tablets has only disadvantages such as reduction in compactability, and delay in disintegration and reduction in active ingredient dissolution ascribable to reduced water-wicking properties. Methods for adding the lubricant include a method of allowing the lubricant to exist only on the surface of tablets using an external lubrication apparatus. However, this method requires a special apparatus and requires preparing a specification from a tableting machine designing stage such that the external lubrication apparatus can be attached later. Thus, the lubricant cannot be easily used without the apparatus. Hence, it is desirable to suppress the spread of the lubricant in the inside of tablets.

In new drug development, the design of formulation or a production method is often changed in preclinical, early clinical, and late clinical phases. The equivalence between a formulation before change and a formulation after change needs to be confirmed with each change, and time and money are used in development. Hence, there is a demand for tablet design that can be manufactured simply by mixing with a drug and compressing directly, and that is less susceptible to the effects of scale-up p.

A co-processed excipient (all-in-one co-processed excipient) is a combination of two or more excipients obtained by physical co-processing and is known to exhibit functions which cannot be achieved by simple mixing of the individual excipients. However, any co-processed excipient capable of sufficiently solving the problems described above has not yet been reported.

Therefore, there is a demand for a pre-formulated co-processed excipient containing pharmaceutical additive components such as diluents, disintegrants, and lubricants, which has the content uniformity and stability of a drug, a suitable disintegrating property, and compactibility, inhibits the influence of a mixing time or a machine of a tableting powder, and enables tablets to be produced by mere mixing of a drug and tableting and the co-processed excipient.

The present applicant has previously proposed spherical co-processed excipients containing anhydrous dibasic calcium phosphate, lactose, and a disintegrant. Examples thereof include a spherical co-processed excipient obtained by spray-drying mannitol, anhydrous dibasic calcium phosphate, lactose, and a disintegrant (Patent Literature 1), a spherical co-processed excipient obtained by spray-drying anhydrous dibasic calcium phosphate, lactose, and a disintegrant (Patent Literature 2), a spherical co-processed excipient obtained by spray-drying anhydrous dibasic calcium phosphate, lactose, and crospovidone (Patent Literature 3), and a granulation product comprising a saccharide, a disintegrant, a excipient, and optionally an inorganic powder, the granulation product being prepared aside from an active ingredient (Patent Literature 4). Thus, in these inventions, the lubricant is mixed with the co-processed excipient during tableting and is not contained in the co-processed excipient.

A spherical composition containing 87% of lactose, 9% of crospovidone, 3% of a polyethylene glycol-polyvinyl alcohol graft polymer, and 1% of sodium stearyl fumarate and having an average particle size of approximately 150 $\mu$m (Non Patent Literature 1) and a composition containing 96% of microcrystalline cellulose, 1.2% of sodium starch glycol, 2% of silicon dioxide, and 0.8% of sodium stearyl fumarate and having an average particle size of approximately 160 $\mu$m (Non Patent Literature 2) have been commercially available as all-in-one co-processed excipients containing a lubricant in recent years.

Citation List

Patent Literature

Patent Literature 1: WO 2005/037254
Patent Literature 2: JP-A-2011-157348
Patent Literature 3: WO 1999/055373
Patent Literature 4: JP-A-2015-78182

Non Patent Literature

Non Patent Literature 1: Kollitab(R) DC87L Technical Information pamphlet
Non Patent Literature 2: PROSOLV(R) EASYtab SP pamphlet

Summary of Invention

Technical Problem

The present invention relates to providing a co-processed excipient which has favorable tabletability, suppresses spread caused by the mixing of a tableting powder, suppresses delay in disintegration and reduction in compactability and hardness, and is used for producing a tablet excellent in content uniformity with a drug, and a tablet comprising the same.

Solution to Problem

The present inventors have conducted studies to solve the problems described above and consequently found that a particulate composition comprising a lubricant, a low soluble saccharide, and anhydrous dibasic calcium phosphate is useful as a co-processed excipient which suppresses spreadability, has favorable tabletability, and is used for producing a tablet excellent in content uniformity with a drug and excellent in disintegrating property and compactability by mere mixing and tableting with a drug.

Specifically, the present invention relates to the following 1) to 27).

1) A particulate composition comprising a lubricant, a low soluble saccharide, and anhydrous dibasic calcium phosphate.
2) The composition according to 1), further comprising a disintegrant.
3) The composition according to 1) or 2), wherein the lubricant is dispersed as lubricant particles having a particle size of 20 $\mu$m or smaller in the composition.
4) The composition according to 1) or 2), wherein an average particle size is from 50 to 200 $\mu$m, a static bulk specific volume is from 1 to 5 mL/cm$^2$, or a Hausner ratio is from 1 to 1.45.
5) The composition according to 1) or 2), wherein a rate of decrease in tablet hardness caused by a mixing time when the composition is mixed and tableted with a drug is 30% or less in 10 minutes and/or 40% or less in 60 minutes based on a mixing time of 0 minutes.
6) The composition according to claim 1 or 2, wherein an acceptance value of content uniformity of a tablet containing 99 mass% of the composition according to 1) or 2) and 1% of a drug in a content uniformity test which abides by the Japanese Pharmacopoeia 18th Edition is 15 or less.

7) The composition according to 1) or 2), wherein the lubricant is one or more members selected from the group consisting of magnesium stearate, calcium stearate, glycerin fatty acid ester, stearic acid, sodium stearyl fumarate, and sucrose fatty acid ester.

8) The composition according to 1) or 2), wherein the lubricant is one or more members selected from the group consisting of magnesium stearate, calcium stearate, and sodium stearyl fumarate.

9) The composition according to 1) or 2), which contains from 0.1 to 5 parts by mass of the lubricant per 100 parts by mass of the particle composition.

10) The composition according to 1) or 2), wherein the lubricant has an average particle size of from 2 to 20 μm.

11) The composition according to 1) or 2), which contains from 10 to 40 parts by mass of the anhydrous dibasic calcium phosphate per 100 parts by mass of the particle composition.

12) The composition according to 1) or 2), which contains from 25 to 35 parts by mass of the anhydrous dibasic calcium phosphate per 100 parts by mass of the particle composition.

13) The composition according to 1) or 2), wherein the anhydrous dibasic calcium phosphate is constituted by primary particles having an average particle size of from 0.1 to 5 μm.

14) The composition according to 1) or 2), wherein the low soluble saccharide is sugar or sugar alcohol having solubility of 50 g or less in 100 g of water at 25°C.

15) The composition according to 1) or 2), wherein the low soluble saccharide is lactose, mannitol, or erythritol.

16) The composition according to 1) or 2), which contains from 50 to 85 parts by mass of the low soluble saccharide per 100 parts by mass of the particle composition.

17) The composition according to 1) or 2), which contains from 60 to 70 parts by mass of the low soluble saccharide per 100 parts by mass of the particle composition.

18) The composition according to 15), wherein a mass ratio between the lactose and the anhydrous dibasic calcium phosphate is from 50:50 to 80:20.

19) The composition according to 2), wherein the disintegrant is a swelling disintegrant.

20) The composition according to 2), wherein the disintegrant is one or more members selected from the group consisting of sodium carboxymethyl starch, croscarmellose sodium, crospovidone, and low substituted hydroxypropylcellulose.

21) The composition according to 2), wherein the disintegrant is contained at from 2 to 20 parts by mass per 100 parts by mass of the particle composition.

22) The composition according to 2), which contains from 3 to 10 parts by mass of the disintegrant per 100 parts by mass in total of the particle composition.

23) A method for producing a particulate composition according to 1), comprising a step of dissolving or dispersing a lubricant, a low soluble saccharide, and anhydrous dibasic calcium phosphate in a solvent to prepare a slurry and a step of removing the solvent from the slurry.

24) A method for producing a particulate composition according to 2), comprising a step of dissolving or dispersing a lubricant, a low soluble saccharide, anhydrous dibasic calcium phosphate and a step of a disintegrant in a solvent to prepare a slurry; and removing the solvent from the slurry.

25) The production method according to 23) or 24), wherein the lubricant is uniformly dispersed in the slurry.

26) A tablet comprising a composition according to 1) or 2) and a drug.

27) A method for producing a tablet, comprising mixing and compacting a composition according to 1) or 2) and a drug.

Advantageous Effects of Invention

By only mixing the particulate composition of the present invention as a co-processed excipient with a drug and tableting the mixture, a tablet of which the delay in disintegration, the reduction in compactability and hardness, and the spreadability are suppressed and which is excellent in the content uniformity with the drug, can be produced.

Brief Description of Drawings

[Figure 1] Figure 1 is a SEM photograph of a particulate composition of Example 1.
[Figure 2] Figure 2 is a SEM-BEX photograph of the inside of the particulate composition of Example 1.
[Figure 3] Figure 3 is a SEM-BEX photograph of the inside of the particulate composition of Example 1.
[Figure 4] Figure 4 is a SEM photograph of a starting material magnesium stearate.
[Figure 5] Figure 5 shows mixing and tableting evaluation: mixing time-hardness.

[Figure 6] Figure 6 shows mixing and tableting evaluation: mixing time-disintegration time.

[Figure 7] Figure 7 is a SEM-BEX photograph of the cross section of a tablet obtained by tableting the particulate composition of Example 1.

[Figure 8] Figure 8 is a SEM-BEX photograph of the cross section of a tablet obtained by tableting a particulate composition of Comparative Example 1 and magnesium stearate.

Description of Embodiments

Hereinafter, the present invention will be described in detail. In the present invention, the application of each pharmaceutical excipient is based on the application of the pharmaceutical excipient described in Japanese Pharmaceutical Excipients, unless otherwise specified.

(Particulate composition)

The particulate composition of the present invention is a spherical composition comprising a lubricant, a low soluble saccharide, and anhydrous dibasic calcium phosphate.

Any of organic lubricants and inorganic lubricants can be used as the lubricant in the particulate composition of the present invention, and an organic lubricant is preferably used. Examples of the organic lubricant include magnesium stearate, calcium stearate, sucrose fatty acid ester, glycerin fatty acid ester, and stearic acid, and sodium stearyl fumarate. Magnesium stearate, calcium stearate, and sodium stearyl fumarate are preferred, and magnesium stearate is more preferred. The average particle size of the lubricant is preferably 20 $\mu$m or smaller, more preferably from 2 to 20 $\mu$m, more preferably from 2 to 20 $\mu$m, further more preferably from 3 to 15 $\mu$m, most preferably from 3 to 10 $\mu$m.

The low soluble saccharide in the particulate composition of the present invention is sugar or sugar alcohol having low solubility in water, for example, solubility of 50 g or less in 100 mL of water at 25°C. Specifically, the low soluble saccharide is lactose, mannitol, or erythritol, preferably lactose or mannitol, further more preferably lactose. In the case of using the particulate composition of the present invention in the production of usual tablets, lactose is most preferred from the viewpoint of easy availability and price. In the case of using the particulate composition of the present invention in the production of orally rapidly disintegrating tablets, mannitol is most preferably used from the viewpoint of taste and texture.

The low soluble saccharide is blended for the purpose of exerting the functions of a diluent and a binder. The low soluble saccharide contains a crystalline moiety and an amorphous moiety. It is considered that the crystalline moiety has action as a diluent while a moiety rich in the amorphous moiety has action as a binder of binding individual components, and action as a diluent.

Anhydrous dibasic calcium phosphate described in the Japanese Pharmacopoeia can be used as the anhydrous dibasic calcium phosphate in the particulate composition of the present invention. Anhydrous dibasic calcium phosphate having good compactability is preferably used. The anhydrous dibasic calcium phosphate to be used is particles constituted by primary particles having an average particle size of from 0.05 to 10 $\mu$m, preferably from 0.1 to 5 $\mu$m, and has a degree of crystallinity of from 0.3 to 0.9, preferably from 0.4 to 0.8. The degree of crystallinity is a ratio to the highest peak of XRD using, as a standard, anhydrous dibasic calcium phosphate sold as a reagent with an average particle size of 50 $\mu$m or larger in crystal particles. A specific commercially available product is Fujicalin(R) (manufactured by Fuji Chemical Industries Co., Ltd.), GS, or GSH (Kyowa Chemical Industry Co., Ltd.), preferably Fujicalin.

The particulate composition of the present invention can optically comprise a disintegrant in the inside of particles thereof.

Examples of the disintegrant include, but are not particularly limited to, crospovidone, carmellose calcium, carmellose, croscarmellose, croscarmellose sodium, low substituted hydroxypropylcellulose, corn starch, potato starch, wheat starch, rice starch, partly pregelatinized starch, pregelatinized starch, and sodium carboxymethyl starch. The disintegrant is preferably crospovidone, carmellose, croscarmellose, low substituted hydroxypropylcellulose, or the like, more preferably crospovidone. One or more of these disintegrants can be used.

The particulate composition of the present invention can comprise appropriate amounts of an excipient and a medicinal ingredient which are generally used in medicaments. One of these components can be used singly, or two or more thereof can be used. In this context, examples of the excipient include diluents, binders, coating agents, glossing agents, colorants, corrigents, sweeteners, and flavors.

The content ratio of the lubricant in the particulate composition of the present invention is from 0.1 to 5 parts by mass, preferably from 0.2 to 4 parts by mass, more preferably from 0.3 to 3 parts by mass, further more preferably from 0.5 to 2 parts by mass, per 100 parts by mass of the composition.

The content ratio of the anhydrous dibasic calcium phosphate in the particulate composition of the present invention is from 10 to 40 parts by mass, preferably from 20 to 40 parts by mass, further more preferably from 25 to 35 parts by mass, per 100 parts by mass of the composition.

The content ratio of the low water-soluble saccharide in the particulate composition of the present invention is from 50 to 85 parts by mass, preferably from 54 to 75 parts by mass, further more preferably from 60 to 70 parts by mass, per 100 parts by mass of the composition.

In the particulate composition of the present invention, for example, the mass ratio between the lactose and the anhydrous calcium phosphate is from 50:50 to 80:20, preferably from 60:40 to 75:25.

In the case of blending a disintegrant, the content ratio of the disintegrant is from 2 to 20 parts by mass, preferably from 3 to 15 parts by mass, further more preferably from 3 to 10 parts by mass, per 100 parts by mass of the composition.

The static bulk specific volume of the particulate composition of the present invention is from 1 to 5 mL/g, preferably from 1.5 to 2.5 mL/g.

The static bulk specific volume is a reciprocal of a bulk density and refers to a volume (mL) per g. This value is obtained by measuring a volume and a mass and dividing the volume by the mass. The bulk density of the Japanese Pharmacopoeia can be measured by the same method as above.

The BET specific surface area of the particulate composition of the present invention is from 1 to 5 $m^2/g$, preferably from 1.5 to 3 $m^2/g$.

The BET specific surface area and pore volume can be calculated by measuring a nitrogen adsorption isotherm with BELSORP-mini II manufactured by MicrotracBEL Corp., followed by analysis with BELMaster Ver 6.3.2.1.

The average particle size of the particulate composition of the present invention is from 40 to 300 $\mu$m, preferably from 50 to 200 $\mu$m, further more preferably from 60 to 150 $\mu$m.

In the present invention, the average particle size is a median size (D50) based on a volume and can be measured using a dry laser diffraction/scattering particle size distribution measurement apparatus. Detailed measurement conditions can include those described in Examples mentioned later.

The Hausner ratio of the particulate composition of the present invention is from 1.00 to 1.45, preferably from 1.08 to 1.34, further more preferably from 1.10 to 1.25.

The Hausner ratio is a value obtained by dividing the static bulk specific volume by a dynamic bulk specific volume and can be determined in accordance with the Hausner ratio measurement method of the Japanese Pharmacopoeia.

The particulate composition of the present invention preferably has a particle structure that components such as a lubricant, a disintegrant, anhydrous dibasic calcium phosphate, and crystalline low soluble saccharide particles are uniformly dispersed without uneven distribution on the surface and in the inside of the particulate composition and cross-linked or bound through the low soluble saccharide. The disintegrant, the anhydrous dibasic calcium phosphate, and the crystalline low soluble saccharide particles constitute such a structure in the inside of the particulate composition, whereby tabletability, a disintegrating property of tablets, and compactability are considered excellent. The lubricant is not unevenly distributed but is uniformly dispersed, particularly, uniformly dispersed on the surface of the particulate composition, as in the inside and is preferably dispersed with a particle size of 20 $\mu$m or smaller, preferably 10 $\mu$m or smaller, further more preferably 5 $\mu$m or smaller. Such a particle size of the lubricant can be confirmed from a SEM photograph and/or a cross-sectional SEM photograph of the particulate composition of the present invention. Partial exposure of the lubricant to the surface is considered to suppress spread during mixing of a tableting powder or in a tableting machine and confer sufficient conpactability between tablet and a die/punch during tableting. A high compression pressure in tableting may destroy the particulate composition at a contact site between the outer periphery of tablets and a die/punch so that the internal lubricant may have a lubricating effect.

In this context, the "uneven distribution" refers to a state in which (1) constituents such as a lubricant have a layered structure in the inside or on the outer periphery of a particle and are contained in a specific layer, and (2) aggregates with an average particle size derived from starting materials of constituents such as a lubricant are contained. On the other hand, the term "uniformly dispersed" is a state in which, without such uneven distribution, individual constituents in the particulate composition are dispersed or cross-linked in the low soluble saccharide by the removal of a solvent while the individual constituent particles maintain their sizes of dispersion treatment in a production solution. The term "uniformly dispersed" refers to a state in which, for example, the anhydrous dibasic calcium phosphate resides with a particle size on the order of mainly from 10 to 40 $\mu$m, not the average particle size of approximately 120 $\mu$m of the starting material, and a state in which the disintegrant maintains its particle size derived from the starting material. In this context, the particle size of each component in the particulate composition can be confirmed by mapping elements in a SEM photograph and a SEM-BEX photograph, for example.

The particulate composition of the present invention suppresses spreadability ascribable to the lubricant when mixed and tableted with a drug. As for the effect of suppressing spreadability, the rate of decrease in tablet hardness from tablet hardness at a mixing time of 0 minutes is 30% or less in a mixing time of 10 minutes and/or 40% or less in 60 minutes, preferably 30% or less in a mixing time of 10 minutes and/or 40% or less in 60 minutes. The amount of a mixed powder to be added based on the capacity of a mixer under measurement conditions differs depending on conditions such as a drug to be mixed or an apparatus, and may not be limited as long as it can be blended, and for example, from 20 to 60% by volume, preferably from 20 to 40% by volume is mixed. Specific conditions are shown in Examples mentioned later. The decrease in disintegration time of tablets by the mixing time varies depending on set hardness and is 15 N or less in a mixing time of 10 minutes and/or 20 N or less in 60 minutes, preferably 10 N or less in a mixing time of 10 minutes and/or 15 N or less in 60 minutes, based on set hardness of 50 N and a mixing time of 0 minutes.

From the particulate composition of the present invention can produce, it is possible to produce a tablet which is excellent in the content uniformity without segregation between the drug and the particulate composition, when mixed and tableted with a drug. When the content of the drug is 5 mass% or less, particularly, 1 mass% or less, with respect to the tablet weight, the problem with the content uniformity are likely to occur, which poses a challenge in direct compresson. However, use of the particulate composition of the present invention can prevent such a problem.

The content uniformity when the particulate composition is mixed and tableted with a drug is 15 or less, preferably 10 or less, more preferably 8 or less, further more preferably 6 or less, in terms of, for example, a determined value of the content uniformity measured in accordance with a content uniformity test of the Japanese Pharmacopoeia 18th Edition as to a tablet produced by mixing 99 mass% of the particulate composition with 1 mass% of the drug.

In this context, the acceptance value is a value obtained according to a acceptance value calculation equation represented by the following equation (Expression 1) in the content uniformity test of the Japanese Pharmacopoeia 18th Edition, wherein X is an mean of individual contents indicated by % based on a quantitative value, k is a acceptability constant and is 2.4 when the number of samples is 10, s is sample standard deviation, and M is M = X at $98.5 \leq X \leq 101.5$.

(Formula 1)

$$\text{Determined value} = |M - X| + ks$$

(Method for producing particulate composition)

A method for producing the particulate composition of the present invention comprises the steps of: dissolving or dispersing a lubricant, a low soluble saccharide, and anhydrous dibasic calcium phosphate in a solvent to prepare a slurry; and removing the solvent from the slurry.

Specifically, the particulate composition of the present invention is produced as spherical particles by dissolving or dispersing a lubricant, a low soluble saccharide, anhydrous dibasic calcium phosphate, and optionally other pharmaceutical excipients in a solvent to prepare a spray liquid (slurry), and instantly removing the solvent by spraying into an air current.

Spray drying, a fluidized bed, or a tumbling bed can be used in a production apparatus, and spray drying is preferably performed because large-scale continuous production can be carried out. When drying is further necessary after granulation, the granulation product can be dried by a usual drying method such as tray drying or fluidized-bed drying so as to attain a desired moisture content. After drying, the particle size can be adjusted by particle size regulation and disintegration.

A water-soluble solvent can be used as the solvent. The solvent is, for example, water, ethanol, methanol, propanol, or acetone, preferably water.

Specific conditions for the spray drying are, for example, inlet temperatures at from 100 to 240°C and outlet temperatures at from 70 to 140°C. A pressurization nozzle or a rotary atomizer can be used as a spraying apparatus, and pressurization conditions or the number of rotations can be set by a routine method depending on a desired particle size.

The particulate composition of the present invention requires uniformly dispersing a lubricant, anhydrous calcium phosphate, lactose, and a disintegrant. The spray liquid is prepared by appropriately setting dispersion, stirring rate, temperature, time, and concentration, particularly, under conditions which disperse the lubricant without liberation. The viscosity of the spray liquid is preferably a higher viscosity within a viscosity range which permits spraying, because the dispersibility of insoluble matter such as a lubricant is enhanced and energy for solvent removal is low.

(Tablet comprising particulate composition)

The particulate composition of the present invention can be puduced by mixing and tableting with an active ingredient (drug) alone. The amount of the active ingredient blended can be appropriately set depending on the characteristics of the active ingredient and is, for example, from 0.0001 to 90 parts by mass of the active ingredient per 100 by mass of the tablet formulation. When the active ingredient is 3 parts by mass or less, the active ingredient can be triturated with the particulate composition of the present invention, and then further mixed with the particulate composition of the present invention, followed by tableting. The tablet may be used as a plain tablet or may be film-coated. The tablet thus produced is excellent in compactability while delay in disintegration is suppressed and decrease in tablet hardness is also suppressed.

In order to possess desired characteristics, pharmaceutical additive components which may be usually used can be mixed and tableted in addition to the particulate composition of the present invention and the active ingredient. Examples of the pharmaceutical additive component include colorants, light shielding agents, sweeteners, stabilizers, and disintegrants.

Examples of the colorant include Food Blue No. 1, Food Blue No. 2, Food Yellow No. 4, Food Red No. 2, Food Red No. 3, Food Blue No. 1 aluminum lake, Food Blue No. 2 aluminum lake, Food Red No. 2 aluminum lake, iron sesquioxide (red), titanium oxide, yellow iron sesquioxide, caramel, and talc.

Examples of the light shielding agent include titanium oxide, calcium carbonate, zinc oxide, talc, iron oxide such as yellow iron sesquioxide, iron sesquioxide, and black iron oxide, Food Yellow No. 5, and Food Red No. 102 and preferably include titanium oxide and calcium carbonate.

Examples of the sweetener include one or more sweeteners selected from the group consisting of table sugar, oligosaccharide, maltitol, erythritol, sorbitol, xylitol, aspartame, acesulfame potassium, sucralose, and stevia.

Examples of the disintegrant include starch such as corn starch and potato starch, partly pregelatinized starch, sodium carboxymethyl starch, carmellose, carmellose calcium, croscarmellose sodium, crospovidone, low substituted hydroxypropylcellulose, crystalline cellulose, and hydroxypropyl starch.

Examples of the fluidizer include talc, aqueous silicon dioxide, light anhydrous silicic acid, magnesium aluminometasilicate, synthetic aluminum silicate, heavy anhydrous silicic acid, magnesium hydroxide-aluminium hydroxide co-precipitates, stearic acid, calcium stearate, and magnesium stearate.

Examples

Hereinafter, the present invention will be described in more detail with reference to Examples, Comparative Examples, and Test Examples. However, the present invention is not limited by these examples. Samples obtained in Examples were evaluated by the following methods.

1. The average particle size was measured using a laser diffraction/scattering particle size distribution measurement apparatus MT3300EX II manufactured by MicrotracBEL Corp. and analyzed using DMS2 Ver 11.1.0-257F2 manufactured by MicrotracBEL Corp. Measurement conditions were as follows: particle permeability: transmission, refractive index of particles; 1.81, particle shape: non-spherical, solvent: nitrogen, and refractive index of solvent: 1.00.

2. The static bulk specific volume was determined according to $V_0/W$ wherein $V_0$ represents a volume, and W represents a sample weight, by inserting a glass tube to a 100 ml measuring cylinder, placing a sample in the glass tube with a funnel so as to attain a volume of from 90 to 100 ml, gently pulling the glass tube out thereof, and flattening the surface of the sample.

3. The Hausner ratio was calculated according to $V_0/V_f$ by measuring the static bulk specific volume $V_0$ and a dynamic bulk specific volume $V_f$. The dynamic bulk specific volume $V_f$ was determined according to $V_f/W$ from a volume $V_f$ when the sample having the determined static bulk specific volume was tapped at a rate of 100 taps/250 seconds from a height of 4 cm.

4. The content uniformity abided by the content uniformity test of the Japanese Pharmacopoeia 18th Edition, and a acceptability constant when the number of samples, n, was 10 was used.

(Example 1)

[Production of particulate composition]

1 part by mass of magnesium stearate, 30 parts by mass of anhydrous dibasic calcium phosphate, 70 parts by mass of lactose hydrate, and 8 parts by mass of crospovidone were dissolved in and mixed with purified water to prepare a spray liquid at room temperature (from 20 to 25°C). This spray liquid was spray-dried using a spray dryer to obtain a particulate composition having an average particle size of approximately 100 μm, a static bulk specific volume of 1.71 mL/g, and a Hausner ratio of 1.18.

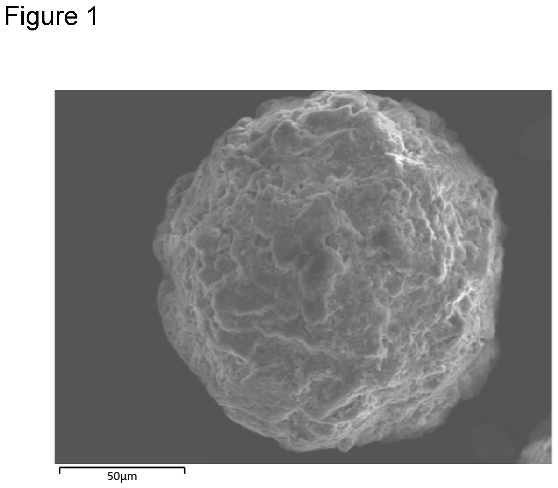

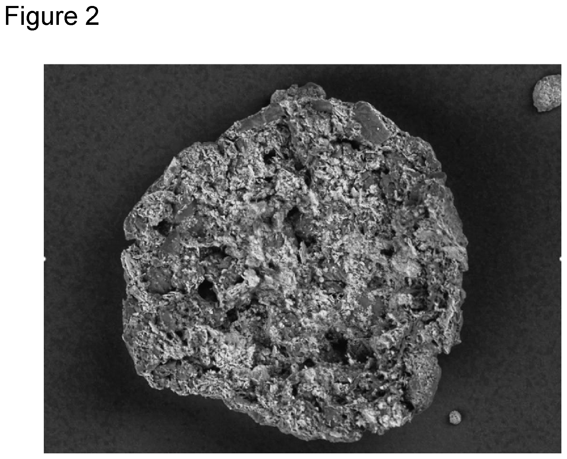

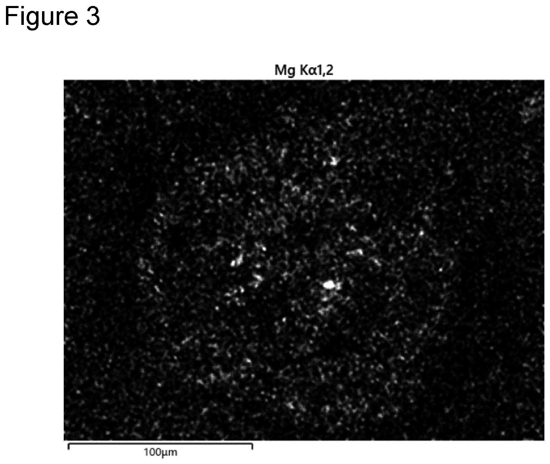

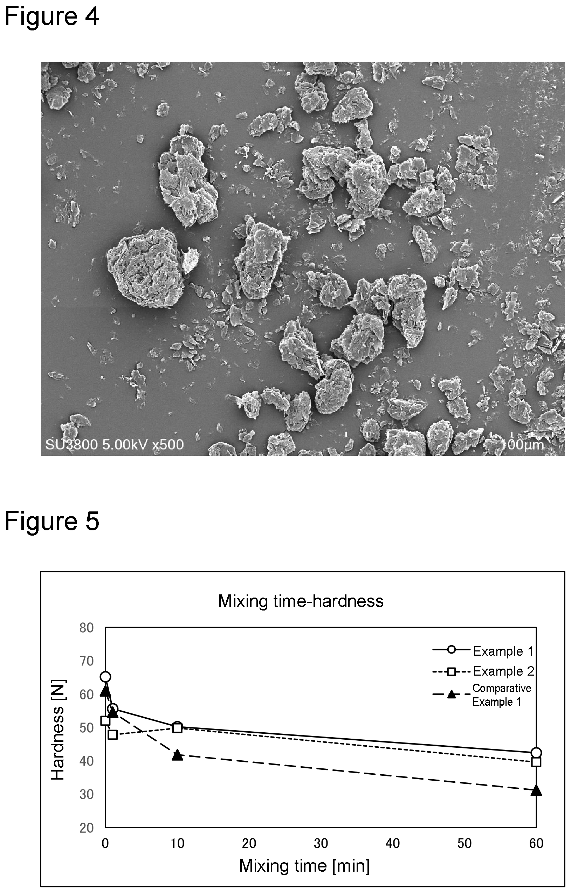

Figure 1 shows a SEM photograph of the particulate composition. Figure 2 shows a SEM-BEX photograph of the inside of the particulate composition. Figure 3 shows a Mg distribution in the SEM-BEX photograph. Figure 4 shows a SEM photograph of the starting material magnesium stearate. It was confirmed that the starting material magnesium stearate had aggregated particles of from 10 to 40 μm, and from the Mg distribution in the SEM-BEX photograph, magnesium stearate formed neither aggregates nor a specific layer, i.e., was found to be uniformly dispersed without uneven distribution and to have a size of 20 μm or smaller at maximum.

The anhydrous dibasic calcium phosphate used was Fujicalin(R) SG manufactured by Fuji Chemical Industries Co., Ltd. (particulate granulation product of anhydrous calcium phosphate having a primary particle size of from 0.1 to 5 μm).

(Example 2)

A particulate composition was obtained under the conditions of Example 1 except that the lubricant was changed to sodium stearyl fumarate.

(Comparative Example 1)

A particulate composition was obtained under the conditions of Example 1 except that magnesium stearate was excluded.

[Mixing and tableting evaluation]

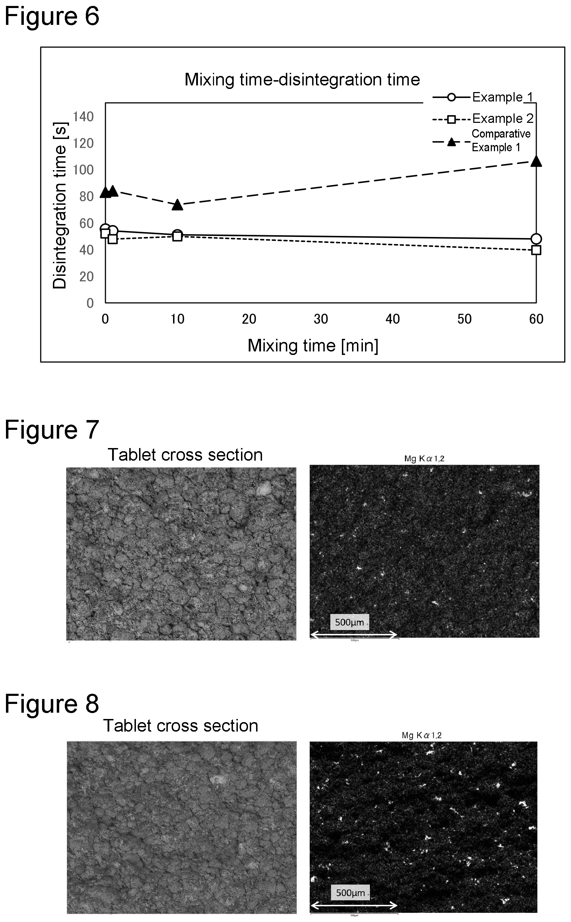

3 kg of a mixed powder of 99.1% of the granulation product of Example 1 or 2 or the granulation product of Comparative Example 1 and 0.9% of magnesium stearate was added to a 20 L tumbler mixer, mixed up to 60 minutes, and then tableted using a rotary tableting machine (manufactured by Kikusui Seisakusho Ltd., VIRGO 0518SS) under the following settings: 8$\phi$12R, the number of rotations: 10 rpm, set hardness: 5 N, and tablet weight: 200 mg. The results are shown in Tables 1, 2, and 3 and Figures 5 and 6.

[Table 1]

| Mixing time and hardness | | | | |
|---|---|---|---|---|
| | Mixing time [min] | | | |
| | 0 | 1 | 10 | 60 |
| Example 1 | 65.2 | 55.6 | 50.2 | 42.4 |
| Example 2 | 52.0 | 47.8 | 49.8 | 39.6 |
| Comparative Example 1 | 61.0 | 54.6 | 41.8 | 31.2 |
| Numeric values represent hardness [N]. | | | | |

[Table 2]

| Mixing time and rate of decrease in hardness | | | | |
|---|---|---|---|---|
| | Mixing time [min] | | | |
| | 0 | 1 | 10 | 60 |
| Example 1 | 0 | 14.7 | 23.0 | 35.0 |
| Example 2 | 0 | 8.1 | 4.2 | 23.8 |
| Comparative Example 1 | 0 | 10.5 | 31.5 | 48.9 |
| Rate of decrease [%] when hardness at a mixing time of 0 minutes is defined as 100 | | | | |

[Table 3]

| Mixing time and disintegration time | | | | |
|---|---|---|---|---|
| | Mixing time [min] | | | |
| | 0 | 1 | 10 | 60 |
| Example 1 | 55 | 54 | 51 | 48 |
| Example 2 | 52 | 48 | 50 | 40 |
| Comparative Example 1 | 93 | 84 | 74 | 106 |
| Numeric values represent time [sec] | | | | |

The tablets produced using the particulate compositions of Examples 1 and 2 had no change in the tablet disintegration time caused by the mixing time, and the decrease in the hardness of the tablets was suppressed. By contrast, in the tablets prepared in Comparative Example 1, delay in disintegration time occurred, and the hardness was largely decreased. That is, the particulate composition of the present invention sufficiently suppressed spreadability and also had favorable tabletability.

Figures 7 and 8 show SEM-BEX photographs and SEM-BEX (Mg) photographs of the tablet cross sections of the tablet obtained from the granulation product of Example 1 (Example 1 tablet) and the tablet obtained from 99.1% of the granulation product of Comparative Example 1 and 0.9% of magnesium stearate (Comparative Example 1 tablet), respectively. The lubricant on the granulation product surface in the tablet cross section in Example 1 had a size of 20 μm or smaller at maximum. More particles of magnesium stearate were present on the tablet cross section of the Comparative Example 1 tablet, i.e., the surface of the granulation product of Comparative Example 1, than on the surface of the granulation product in the Example 1 tablet, and furthermore, the particle size of Comparative Example 1 was large. This is presumably because the original particle surface was rich in magnesium stearate, which adhered to the surface with the size substantially derived from the starting material.

(Comparative Example 2)

The granulation product of Comparative Example 1 was tableted using a rotary tableting machine (manufactured by Kikusui Seisakusho Ltd., VIRGO 0518SS) under the following settings: 8φ12R, the number of rotations: 10 rpm, set hardness: 5 N, and tablet weight: 200 mg. Sufficient tablets could not be obtained due to sticking to a die/punch.

(Examples 3 to 6)

Particulate compositions were obtained in accordance with the method of Example 1 using the amounts of magnesium stearate, anhydrous dibasic calcium phosphate, lactose hydrate, and crospovidone blended in Table 4. Table 5 shows the physical property evaluation of the particulate compositions, and Table 6 shows results of the hardness and the disintegration time.

[Table 4]

| Amount of each component blended | | | | |
|---|---|---|---|---|
| | Example 3 | Example 4 | Example 5 | Example 6 |
| Magnesium stearate | 0.0075 | 0.01 | 0.01 | - |
| Sodium stearyl fumarate | - | - | - | 0.005 |
| Anhydrous dibasic calcium phosphate | 0.35 | 0.25 | 0.25 | 0.3 |
| Lactose hydrate | 0.5425 | 0.64 | 0.675 | 0.63 |
| Crospovidone | 0.1 | 0.1 | 0.065 | 0.065 |

[Table 5]

| Physical property evaluation of particulate composition | | | | |
|---|---|---|---|---|
| | Example 3 | Example 4 | Example 5 | Example 6 |
| Static bulk specific volume [L/g] | 1.73 | 1.70 | 1.68 | 1.70 |
| Hausner ratio | 1.18 | 1.24 | 1.28 | 1.21 |

[Table 6]

| Evaluation of hardness and tabletability | | | | |
|---|---|---|---|---|
| | Example 3 | Example 4 | Example 5 | Example 6 |
| Hardness [N] | 91.6 | 71.6 | 83.6 | 89.6 |
| Disintegration time [sec] | 50 | 44 | 37 | 30 |

The particulate compositions of the present invention in Examples 3 to 6 had sufficiently favorable hardness and disintegrating property in tablet production.

(Examples 7 and 8)

99 mass% of the particulate composition of Example 1 was mixed with 1 mass% of rebamipide or 1 mass% of loxoprofen sodium hydrate and then tableted using a rotary tableting machine under the following settings: 8φ12R, set hardness: 5 N, the number of rotations: 30 rpm, and tablet weight: 200 mg. Table 7 shows results of the content uniformity, the hardness, and the disintegrating property.

[Table 7]

| Formulation evaluation | | Example 7 | Example 8 |
|---|---|---|---|
| | | Rebamipide | Loxoprofen sodium hydrate |
| Hardness [N] | | 50.4 | 51.3 |
| Disintegration time [sec] | | 101.00 | 94.67 |
| Content uniformity | Drug content [%] | $101.1 \pm 1.14$ | $99.3 \pm 1.66$ |
| | Determined value | 2.7 | 4.0 |

The tablets containing the particulate composition of the present invention and the active ingredient had sufficiently favorable hardness and disintegrating property. The determined value of the content uniformity was 15.0 or less, and it was evaluated as acceptable. Thus, the particulate composition of the present invention had excellent content uniformity, although the drug content was low at a level of 1%.

(Example 9)

90 parts by mass of the particulate composition of Example 1 was mixed with 10 parts by mass of acetaminophen and then tableted using a rotary tableting machine under the following settings: 8φ12R, set hardness: 5 N, the number of rotations: 30 rpm, and tablet weight: 200 mg to obtain tablets having tablet hardness of 45.0 N and a disintegration time of 88.0 seconds.

**Claims**

1.   A particulate composition comprising a lubricant, a low soluble saccharide, and anhydrous dibasic calcium phosphate.

2.   The composition according to claim 1, further comprising a disintegrant.

3.   The composition according to claim 1 or 2, wherein the lubricant is dispersed as lubricant particles having a particle size of 20 μm or smaller on the surface and in the inside of the composition.

4.   The composition according to claim 1 or 2, wherein an average particle size is from 50 to 200 μm, a static bulk specific volume is from 1 to 5 mL/g, or a Hausner ratio is from 1 to 1.45.

5.   The composition according to claim 1 or 2, wherein a rate of decrease in tablet hardness caused by a mixing time when the composition is mixed and tableted with a drug is 30% or less in 10 minutes and/or 40% or less in 60 minutes based on a mixing time of 0 minutes.

6.   The composition according to claim 1 or 2, wherein an acceptance value of content uniformity of a tablet containing 99 mass% of the composition according to claim 1 or 2 and 1% of a drug in a content uniformity test which abides by the Japanese Pharmacopoeia 18th Edition is 15 or less.

7.   The composition according to claim 1 or 2, wherein the lubricant is one or more selected from the group consisting of magnesium stearate, calcium stearate, glycerin fatty acid ester, stearic acid, sodium stearyl fumarate, and sucrose fatty acid ester.

8. The composition according to claim 1 or 2, wherein the lubricant is one or more selected from the group consisting of magnesium stearate, calcium stearate, and sodium stearyl fumarate.

9. The composition according to claim 1 or 2, which contains from 0.1 to 5 parts by mass of the lubricant per 100 parts by mass of the particle composition.

10. The composition according to claim 1 or 2, wherein the lubricant has an average particle size of from 2 to 20 $\mu$m.

11. The composition according to claim 1 or 2, wherein the anhydrous dibasic calcium phosphate is contained at from 10 to 40 parts by mass per 100 parts by mass of the particle composition.

12. The composition according to claim 1 or 2, wherein the anhydrous dibasic calcium phosphate is contained at from 25 to 35 parts by mass per 100 parts by mass of the particle composition.

13. The composition according to claim 1 or 2, wherein the anhydrous dibasic calcium phosphate is constituted by primary particles having an average particle size of from 0.1 to 5 $\mu$m.

14. The composition according to claim 1 or 2, wherein the low soluble saccharide is sugar or sugar alcohol having solubility of 50 g or less in 100 g of water at 25°C.

15. The composition according to claim 1 or 2, wherein the low soluble saccharide is lactose, mannitol, or erythritol.

16. The composition according to claim 1 or 2, which contains from 50 to 85 parts by mass of the low soluble saccharide per 100 parts by mass of the particle composition.

17. The composition according to claim 1 or 2, which contains from 60 to 70 parts by mass of the low soluble saccharide per 100 parts by mass of the particle composition.

18. The composition according to claim 15, wherein a mass ratio between the lactose and the anhydrous dibasic calcium phosphate is from 50:50 to 80:20.

19. The composition according to claim 2, wherein the disintegrant is a swelling disintegrant.

20. The composition according to claim 2, wherein the disintegrant is one or more selected from the group consisting of sodium carboxymethyl starch, croscarmellose sodium, crospovidone, and low substituted hydroxypropylcellulose.

21. The composition according to claim 2, wherein the disintegrant is contained at from 2 to 20 parts by mass per 100 parts by mass of the particle composition.

22. The composition according to claim 2, which contains from 3 to 10 parts by mass of the disintegrant per 100 parts by mass in total of the particle composition.

23. A method for producing the particulate composition according to claim 1, comprising a step of dissolving or dispersing a lubricant, a low soluble saccharide, and anhydrous dibasic calcium phosphate in a solvent to prepare a slurry and a step of removing the solvent from the slurry.

24. A method for producing the particulate composition according to claim 2, comprising the steps of dissolving or dispersing a lubricant, a low soluble saccharide, anhydrous dibasic calcium phosphate, and a disintegrant in a solvent to prepare a slurry a step of and removing the solvent from the slurry.

25. The production method according to claim 23 or 24, wherein the lubricant is uniformly dispersed in the slurry.

26. A tablet comprising the composition according to claim 1 or 2 and a drug.

27. A method for producing a tablet, comprising mixing and compacting the composition according to claim 1 or 2 and a drug.

Figure 1

Figure 2

Figure 3

Mg Kα1,2

100μm

Figure 4

Figure 5

# Figure 6

# Figure 7

Tablet cross section

# Figure 8

Tablet cross section

# EP 4 772 174 A1

| | INTERNATIONAL SEARCH REPORT | International application No. |
|---|---|---|
| | | **PCT/JP2024/030882** |

**A. CLASSIFICATION OF SUBJECT MATTER**

*A61K 9/14*(2006.01)i; *A61K 9/20*(2006.01)i; *A61K 47/02*(2006.01)i; *A61K 47/12*(2006.01)i; *A61K 47/14*(2017.01)i;
*A61K 47/26*(2006.01)i; *A61K 47/32*(2006.01)i; *A61K 47/36*(2006.01)i; *A61K 47/38*(2006.01)i
FI:   A61K9/14; A61K9/20; A61K47/12; A61K47/14; A61K47/26; A61K47/02; A61K47/36; A61K47/32; A61K47/38

According to International Patent Classification (IPC) or to both national classification and IPC

**B. FIELDS SEARCHED**

Minimum documentation searched (classification system followed by classification symbols)

A61K9/14; A61K9/20; A61K47/02; A61K47/12; A61K47/14; A61K47/26; A61K47/32; A61K47/36; A61K47/38

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2013/117963 A1 (PIRAMAL ENTERPRISES LIMITED) 15 August 2013 (2013-08-15) examples 2-4 | 1-27 |
| X | JP 2008-143853 A (NICHI-IKO PHARMACEUTICAL CO., LTD.) 26 June 2008 (2008-06-26) example 1 | 1-27 |
| A | WO 2005/037254 A1 (FUJI CHEMICAL INDUSTRY CO., LTD.) 28 April 2005 (2005-04-28) entire text | 1-27 |
| A | JP 2009-196940 A (TAKADA SEIYAKU KK) 03 September 2009 (2009-09-03) entire text | 1-27 |
| A | WO 2009/113522 A1 (ASKA PHARMACEUTICAL CO., LTD.) 17 September 2009 (2009-09-17) entire text | 1-27 |

☐ Further documents are listed in the continuation of Box C.      ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 November 2024** | **26 November 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
**Information on patent family members**

International application No.

**PCT/JP2024/030882**

| Patent document cited in search report | | | Publication date (day/month/year) | Patent family member(s) | | | Publication date (day/month/year) |
|---|---|---|---|---|---|---|---|
| WO | 2013/117963 | A1 | 15 August 2013 | (Family: none) | | | |
| JP | 2008-143853 | A | 26 June 2008 | (Family: none) | | | |
| WO | 2005/037254 | A1 | 28 April 2005 | US | 2007/0275058 | A1 | |
| | | | | entire text | | | |
| | | | | EP | 1674083 | A1 | |
| | | | | entire text | | | |
| JP | 2009-196940 | A | 03 September 2009 | (Family: none) | | | |
| WO | 2009/113522 | A1 | 17 September 2009 | US | 2011/0020455 | A1 | |
| | | | | entire text | | | |
| | | | | EP | 2251038 | A1 | |
| | | | | entire text | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005037254 A
- JP 2011157348 A
- WO 1999055373 A
- JP 2015078182 A

**Non-patent literature cited in the description**

- *Kollitab(R) DC87L Technical Information pamphlet*
- *PROSOLV(R) EASYtab SP pamphlet*
- Japanese Pharmacopoeia
- the Japanese Pharmacopoeia